# EUROPÄISCHE PATENTSCHRIFT

(11) **EP 0 432 086 B1**
(45) Veröffentlichungstag und Bekanntmachung des Hinweises auf die Patenterteilung: **04.10.1995**
(21) Anmeldenummer: 90730018.0
(22) Anmeldetag: 07.12.1990
(51) Int. Cl.: H01J 61/33, H01J 61/72, A23B 7/015, A23L 3/28, A61L 2/10

(54) **UV-Lichtquelle**
UV light source
Source de lumière UV

(30) Priorität: 07.12.1989 DE 3940499
(43) Veröffentlichungstag der Anmeldung: 12.06.1991
(73) Patentinhaber: Wedeco Umwelttechnologie Wasser-Boden-Luft GmbH, D-32051 Herford (DE)
(72) Erfinder: Wedekamp, Horst, W-4900 Herford (DE)
(74) Vertreter: Thömen, Uwe, Dipl.-Ing.

(56) Entgegenhaltungen:
- DE-A- 3 910 957
- FR-A- 802 714
- GB-A- 1 008 861
- US-A- 2 482 421

## Beschreibung

Die Erfindung betrifft ein UV-Lichtquelle gemäß dem Oberbegriff des Patentanspruchs 1.

Es sind bereits UV-Lichtquellen in Form von Hg-Niederdruckdampflampen bekannt. Das Maximum des Emissionsspektrums dieser Lampen liegt bei etwa 254 nm. Deshalb sind diese Lampen besonders zur photochemischen Inaktivierung bzw. Entkeimung von Mikroorganismen geeignet. Dabei wird eine hohe Strahlungsdichte (UV-Strahlungsleistung pro durchstrahlter Volumeneinheit) angestrebt, um eine wirksame Entkeimung zu erreichen.

Zur Erzielung hoher Strahlungsdichten sind bereits Flachstrahler entwickelt worden (vgl. DE-PS 28 25 0̸18), mit denen im Vergleich zu den herkömmlichen Rundstrahlern eine wesentlich höhere Strahlungsleistung erzielt werden kann. Jedoch hat sich in der Praxis gezeigt, daß auch die Flachstrahler den Anforderungen hinsichtlich der Leistung häufig nicht genügen. Einer Leistungssteigerung durch größere Abmessungen des Strahlers sind dabei durch die jeweiligen Einsatzbedingungen wie etwa der Größe der Entkeimungskammer Grenzen gesetzt. Im übrigen muß auch auf die Druckfestigkeit des Lampenkörpers geachtet werden, die erforderlich ist, weil im Lampeninnenraum annähernd ein Vakuum herrscht. Deshalb sind relativ dicke Wandstärken für den Lampenkörper erforderlich. Je größer aber die Wandstärke ist, um so mehr erhöht sich auch die dadurch bedingte Absorbtion der UV-Strahlung, was der angestrebten Erhöhung einer Strahlungsleistung entgegensteht.

Schließlich sind einer Erhöhung der Strahlungsleistung in der Praxis auch dadurch Grenzen gesetzt, daß sich keine hinreichende Kühlung des Lampenkörpers mehr garantieren läßt.

Der Erfindung liegt die Aufgabe zugrunde, unter Vermeidung der beschriebenen Nachteile eine UV-Lichtquelle zur Verfügung zu stellen, mit der - verglichen mit bekannten UV-Strahlern - bei gleichbleibender Baugröße eine höhere Strahlungsdichte erreicht werden kann, und zwar unter Berücksichtigung der erforderlichen Druckfestigkeit des Lampenkörpers und einer hinreichenden Kühlung.

Dieses Ziel erreicht die Erfindung bei der im Oberbegriff des Patentanspruchs 1 angegebenen UV-Lichtquelle durch die Merkmale des kennzeichnenden Teils des Anspruchs 1.

Durch die erfindungsgemäße Profilierung des Lampenkörpers wird die Druckfestigkeit des Lampenkörpers gegenüber dem Vakuum im Lampeninnenraum erhöht. Daraus ergibt sich, daß die Wandstärke des Lampenkörpers wesentlich reduziert werden kann. Bei gleicher Druckfestigkeit resultieren aus einer dünneren Wandstärke des Lampenkörpers mehrere Vorteile:
a) geringere Strahlungsverluste durch Absorbtion,
b) verbesserte Kühlung durch höhere Leitfähigkeit,
c) Betrieb mit höheren elektrischen Leistungen aufgrund der besseren Kühleigenschaften.

Durch Messungen konnte bestätigt werden, daß die UV-Emission eines UV-Strahlers infolge der obigen Vorteile gemäß a) und b) um bis zu 10̸ % und gemäß dem oben genannten Vorteil c) nochmals um bis zu 10̸ % gesteigert wird, so daß insgesamt bei gleichbleibender Baugröße eine wesentlich höhere Strahlungsdichte erreicht werden kann.

Zudem erlaubt die Profilierung der Oberfläche des Lampenkörpers in vorteilhafter Weise auch die Erzeugung einer im wesentlichen gerichteten Strahlung. Somit ist es möglich, die Strahlungsverteilung der UV-Lichtquelle in vorteilhafter Weise in Übereinstimmung mit der Strömungsverteilung bzw. der Strömungsrichtung eines zu entkeimenden Mediums auszurichten. Da sich die Strahlungsverteilung ideal in Übereinstimmung mit der Strömungsverteilung bringen läßt, ergibt sich eine wesentlich wirksamere Entkeimung.

Der Erfindungsgedanke wird nicht verlassen, wenn andere als in den Ansprüchen dargelegte geeignete Profilierungen, z.B. punktförmige Einziehungen, Riffelungen usw. vorgesehen werden.

In vorteilhafter Ausgestaltung der Erfindung wird die Profilierung der oberfläche des Lampenkörpers mittels parallel zueinander angeordneter Rundstrahler realisiert, deren Querschnitte sich unter Bildung eines einheitlichen Druckraumes durchdringen. Von besonderem Vorteil ist dabei eine Ausgestaltung, bei der die Mittelpunkte der Rundstrahlerquerschnitte auf einer Linie liegen.

Durch diese Maßnahme ist es möglich, eine im wesentlichen gerichtete Strahlung der UV-Lichtquelle zu gewährleisten.

Noch besser läßt sich eine gerichtete Strahlung erzielen, wenn der Lampenkörper in einer anderen Ausgestaltung der Erfindung als ein an sich bekannter Flachstrahler ausgebildet ist und die Profilierung seiner Oberfläche aus mindestens einer sich in Längsrichtung erstreckenden Vertiefung besteht.

Weitere vorteilhafte Ausgestaltungen der Erfindung ergeben sich aus den Unteransprüchen und der nachfolgenden Beschreibung. Anhand der in der Zeichnung dargestellten Ausführungsbeispiele wird die Erfindung näher erläutert. Es zeigen:
- Fig. 1: eine perspektivische Ansicht einer Ausführungsform der Erfindung,
- Fig. 2: einen Querschnitt der UV-Lichtquelle gemäß Fig. 1 mit angedeutetem Strahlungsverlauf der Strahlungslinien,
- Fig. 3: einen Querschnitt einer zweiten Ausführungform der Erfindung, und
- Fig. 4 u. 5: weitere Querschnittsansichten gemäß gemäß weiteren Ausführungsformen der Erfindung mit angedeutetem Strahlungsverlauf der Strahlungslinien.

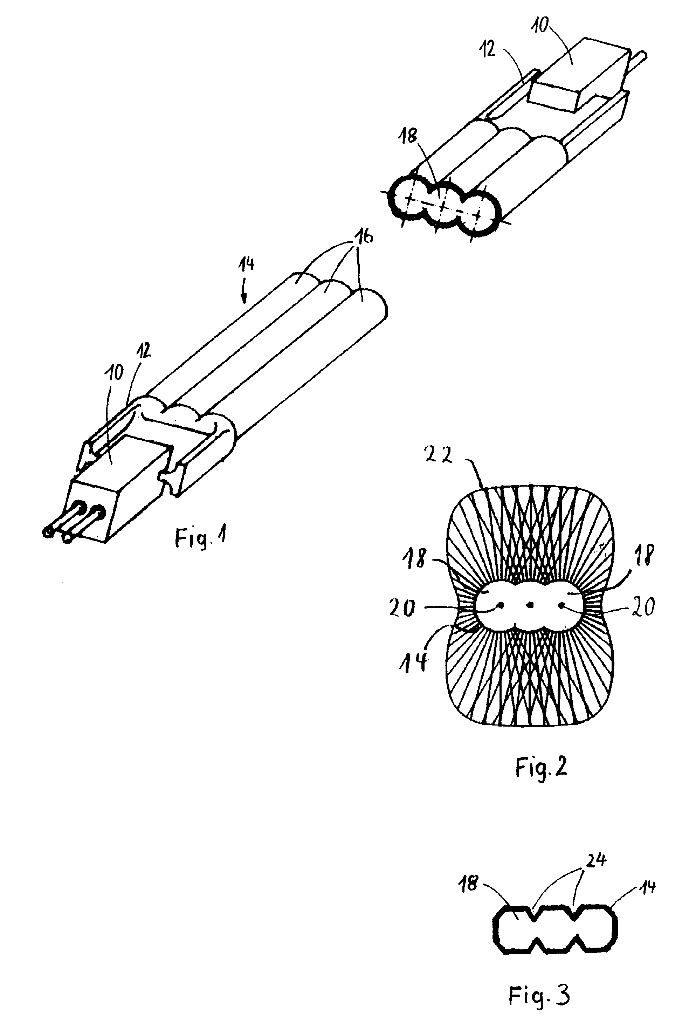

Die in Fig. 1 und 2 dargestellte erste Ausführungsform einer UV-Lichtquelle besteht aus den üblichen Stromzuführungen 10̸ und Halterungen 12 sowie einem Lampenkörper 14. Der Lampenkörper 14 ist dem Prinzip nach eine aus parallel zueinander angeordneten Rundstrahlern 16 zusammengesetzter Flachstrahler, wobei sich die Querschnitte der Rundstrahler 16 unter Bildung eines einheitlichen Dampfdruckraumes 18 durchdringen.

Aufgrund der damit erreichten Profilierung der Oberfläche des Lampenkörpers 14 wird im Vergleich mit herkömmlichen Flachstrahlern eine Vergrößerung der abstrahlenden Oberfläche erreicht, woraus - wie aus dem in Fig. 2 prinzipiell dargestellten Strahlungsverlauf 22 hervorgeht - eine höhere Strahlungsdichte resultiert.

Bei der in Fig. 3 dargestellten Ausführungsform erfolgt bei einem Flachstrahlerquerschnitt die Oberflächenvergrößerung durch jeweils zwei sich über die gesamte Länge der beiden Breitseiten des Lampenkörpers 14 erstreckende und diametral gegenüberliegende Dreiecksnuten 24. Diese Dreiecksnuten 24 stellen eine Einschnürung des Dampfdruckraumes 18 dar, wodurch die sich üblicherweise etwa zylinderförmige ausbildende Gasentladungssäule seitlich gestreckt wird und zusätzlich zu den bereits genannten Effekten eine höhere Strahlungsdichte in den Seitenbereichen des Strahlers erzielt werden kann.

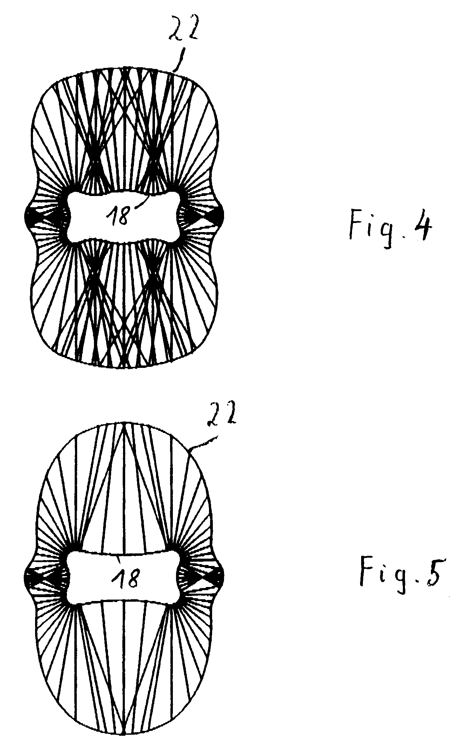

Schließlich zeigen Fig. 4 und 5 noch zwei weitere Ausführungsformen der Erfindung, wobei der prinzipielle Strahlungsverlauf durch entsprechende Linien angedeutet ist.

## Patentansprüche

1. UV-Lichtquelle, die einen für UV-Licht durchlässigen, als Flachstrahler ausgebildeten Lampenkörper aufweist, als Quecksilber-Niederdruckdampflampe ausgebildet und insbesondere für den Einsatz in Entkeimungsanlagen vorgesehen ist, dadurch gekennzeichnet, daß die Oberfläche des Lampenkörpers (14) profiliert, jedoch nicht mit sternförmigem Querschnitt, ausgebildet ist.

2. UV-Lichtquelle nach Anspruch 1, dadurch gekennzeichnet, daß die Profilierung der Oberfläche des Lampenkörpers (14) mittels parallel zueinander angeordneter Rundstrahler (16) realisiert ist, deren Querschnitte sich unter Bildung eines einheitlichen Dampfdruckraumes (18) durchdringen.

3. UV-Lichtquelle nach Anspruch 2, dadurch gekennzeichnet, daß die Mittelpunkte (20̸) der Rundstrahlerquerschnitte auf einer Linie liegen.

4. UV-Lichtquelle nach Anspruch 2, dadurch gekennzeichnet, daß die Mittelpunkte (20̸) der Rundstrahlerquerschnitte auf den Eckpunkten geometrischer Figuren liegen.

5. UV-Lichtquelle nach Anspruch 1, dadurch gekennzeichnet, daß der Lampenkörper (14) als ein an sich bekannter Flachstrahler ausgebildet ist, und die Profilierung seiner Oberfläche aus mindestens einer sich in seiner Längsrichtung erstreckenden Vertiefung (24) besteht.

6. UV-Lichtquelle nach Anspruch 5, dadurch gekennzeichnet, daß die Vertiefung eines Dreiecksnut (24) ist.

## Claims

1. Ultraviolet light source, which comprises a lamp body constructed as a flat-section emitter transparent to UV light, is constructed as a low-pressure mercury vapour lamp and in particular is intended for use in sterilizing plants, characterized in that the surface of the lamp body (14) is of profiled form, but not with a star-shaped cross-section.

2. Ultraviolet light source according to Claim 1, characterized in that the profiling of the surface of the lamp body (14) is realized by means of mutually parallel round emitters (16), the cross-sections of which interpenetrate one another to form one continuous vapour pressure chamber (18).

3. Ultraviolet light source according to Claim 2, characterized in that the centre-points (20) of the round emitter cross-sections lie on one line.

4. Ultraviolet light source according to Claim 2, characterized in that the centre-points (20) of the round emitter cross-sections lie on the corner points of geometrical figures.

5. Ultraviolet light source according to Claim 1, characterized in that the lamp body (14) is constructed as a known flat-section emitter, and the profiling of its surface consists of at least one depression (24) extending in its longitudinal direction.

6. Ultraviolet light source according to Claim 5, characterized in that the depression is a triangular groove (24).

## Revendications

1. Source de lumière UV qui comporte un corps de lampe formant une source plate de rayonnement, sous forme d'une lampe à vapeur de mercure basse pression et, en particulier, prévue pour être utilisée dans des installations de stérilisation, caractérisée en ce que la surface du corps de lampe (14) est profilée, sans avoir pourtant une section transversale en forme d'étoile.

2. Source de lumière UV suivant la revendication 1, caractérisée en ce que le profilage de la surface du corps de lampe (14) est réalisée au moyen de sources rondes de rayonnement (16) parallèles entre elles dont les sections transversales s'interpénètrent en créant un espace à pression de vapeur uniforme.

3. Source de lumière UV suivant la revendication 2, caractérisée en ce que les points centraux (20) des sections des sources rondes sont alignés.

4. Source de lumière UV suivant la revendication 2, caractérisée en ce que les points centraux (20) des sections des sources rondes sont sur les sommets de figures géométriques.

5. Source de lumière UV suivant la revendication 1, caractérisée en ce que le corps de lampe (14) consiste en une source de rayonnement connue en soi et en ce que le profilage de sa surface comporte au moins une rainure (24) dirigée longitudinalement.

6. Source de lumière UV suivant la revendication 5, caractérisée en ce que la rainure est triangulaire.
